(19)

Europäisches
Patentamt
European
Patent Office
Office européen
des brevets

(11) **EP 4 756 008 A1**

(12) **EUROPEAN PATENT APPLICATION**
published in accordance with Art. 153(4) EPC

(43) Date of publication:
**10.06.2026 Bulletin 2026/24**

(21) Application number: **24848634.2**

(22) Date of filing: **16.05.2024**

(51) International Patent Classification (IPC):
***C12M 1/04*** (2006.01)    ***G05D 7/06*** (2006.01)

(52) Cooperative Patent Classification (CPC):
**C12M 1/04; G05D 7/06**

(86) International application number:
**PCT/JP2024/018077**

(87) International publication number:
**WO 2025/027973 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.07.2023 JP 2023123112**

(71) Applicant: **SMC Corporation
Tokyo 104-0031 (JP)**

(72) Inventors:
• **KATSUMATA Koichi
Ibaraki 300-2493 (JP)**
• **OSHIMA Yuta
Ibaraki 300-2493 (JP)**

(74) Representative: **Keil & Schaafhausen
Patentanwälte PartGmbB
Bockenheimer Landstraße 25
60325 Frankfurt am Main (DE)**

(54) **GAS SUPPLY DEVICE**

(57)    A gas supply device (10) for supplying gas to a biological sample comprises: an inlet port (122) through which the gas flows in; an outlet port (124) through which the gas flows out; a discharge port (126) through which the gas is discharged to the outside; a pressure adjustment valve (82) having a main valve (132) that adjusts, according to a pilot pressure, the pressure of the gas flowing through a flow path (150), and an exhaust valve (134) that discharges the gas in the flow path from the discharge port in accordance with the pilot pressure; a pilot pressure adjustment solenoid valve (84) that adjusts the pilot pressure; and a throttle flow path (50) that limits the flow rate of the gas to a predetermined flow rate.

FIG. 1

**Description**

TECHNICAL FIELD

The present invention relates to a gas supply device.

BACKGROUND ART

WO 2018/070464 A1 discloses a pressure-type flow rate control device for supplying a gas used in the manufacture of semiconductors. The pressure-type flow rate control device includes a common inflow port through which the gas flows in from a gas supply source, and a common outflow port through which the gas flows out to a process chamber of a semiconductor manufacturing device. A first flow path and a second flow path are provided between the common inflow port and the common outflow port. Each of the first flow path and the second flow path is provided with a control valve and a throttle portion. The first flow path is used when causing a high flow rate gas to flow, and the second flow path is used when causing a low flow rate gas to flow. The gas flows through only one of the flow paths under the control of the control valve.

SUMMARY OF THE INVENTION

According to the pressure-type flow rate control device disclosed in WO 2018/070464 A1, in the case where the flow rate passing through the throttle portion is a minute flow rate, the gas is likely to remain upstream of the throttle portion. The residual gas may affect the use of the device to which the gas is supplied. For example, a case is assumed in which the pressure-type flow rate control device disclosed in WO 2018/070464 A1 is used to supply a gas such as air to a biological sample in a bioreactor at a minute flow rate. In this case, even after the control valve is closed, the residual gas is continuously output at a minute flow rate for a long time. It is desirable that the residual gas is output quickly.
The present invention has the object of solving the aforementioned problem.
An aspect of the present invention is characterized by a gas supply device that performs gas supply to a biological sample, the gas supply device comprising: a pressure adjusting valve including an inlet port into which a gas from a gas supply source flows, an outlet port from which the gas flows out, a discharge port from which the gas is discharged to an outside of the gas supply device, a main valve configured to adjust, according to a pilot pressure, a pressure of the gas flowing through a flow path configured to allow communication between the inlet port and the outlet port, and an exhaust valve configured to discharge the gas in the flow path from the discharge port according to the pilot pressure; a pilot pressure adjusting solenoid valve configured to adjust the pilot pressure; and a throttle flow path configured to limit a flow rate of the gas flowing out from the outlet port to a predetermined flow rate and output the gas at the predetermined flow rate for the gas supply.
According to the present invention, it is possible to quickly output the gas remaining in the gas supply device including the throttle flow path that limits the flow rate of the gas to a minute flow rate.
The above and other objects, features, and advantages of the present invention will become more apparent from the following description when taken in conjunction with the accompanying drawings, in which a preferred embodiment of the present invention is shown by way of illustrative example.

BRIEF DESCRIPTION OF DRAWINGS

[FIG. 1] FIG. 1 is a diagram illustrating a gas supply device and a control circuit that controls the gas supply device;
[FIG. 2] FIG. 2 is a diagram schematically showing a pressure adjustment unit;
[FIG. 3] FIG. 3 is a diagram showing a throttle laminated plate;
[FIG. 4] FIG. 4 is an enlarged view of the throttle laminated plate;
[FIG. 5] FIG. 5 is a diagram showing a configuration of the throttle laminate plate; and
[FIG. 6] FIG. 6A is a diagram explaining a rectangular cross section of a throttle flow path, FIG. 6B is a diagram showing the rectangular cross section of the throttle flow path, and
FIG. 6C is a diagram explaining the flow path width of the throttle flow path.

DETAILED DESCRIPTION OF THE INVENTION

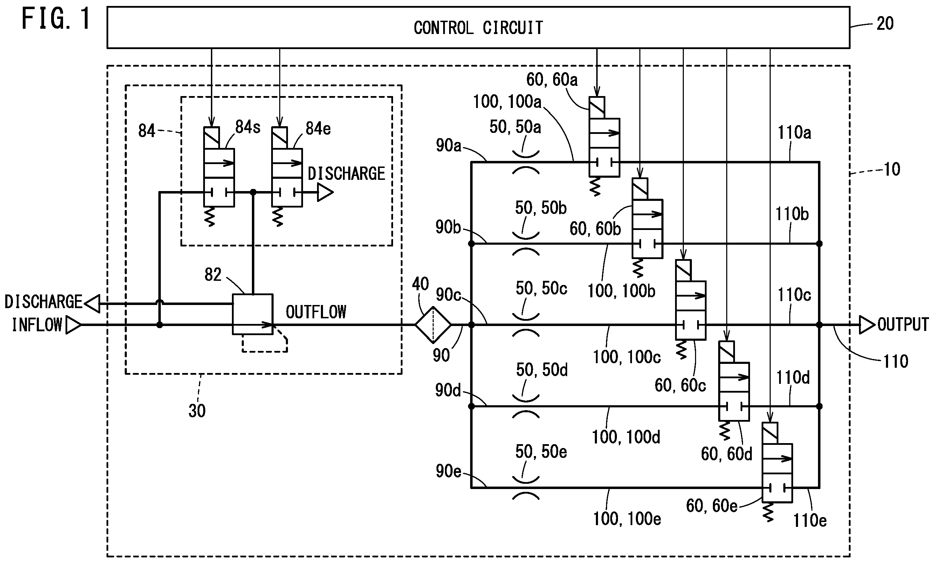

FIG. 1 is a diagram illustrating a gas supply device 10 and a control circuit 20 that controls the gas supply device 10. The gas supply device 10 supplies a gas to a biological sample accommodated in a bioreactor (not shown). The gas supplied to the biological sample is a gas that does not cause a problem even when discharged to the outside of the gas supply device 10, and is, for example, air containing oxygen, nitrogen, and carbon dioxide.

The gas supply device 10 limits the flow rate of the gas flowing therein from a gas supply source (not shown) to a minute flow rate in order to supply the gas to the biological sample. The minute flow rate includes, for example, a flow rate from 0.01 [mL/min] to 1000 [mL/min]. The gas supply device 10 outputs the gas with the limited flow rate to the bioreactor as the gas to be supplied to the biological sample.

The control circuit 20 includes a processor such as a CPU (Central Processing Unit), and a memory such as a RAM (Random Access Memory) and a ROM (Read Only Memory). The control circuit 20 controls the gas supply device 10 based on a command signal from a higher-level device (not shown) by executing a program stored in the memory. The higher-level device is, for example, a PLC (Programmable Logic Controller).

The gas supply device 10 includes a pressure adjustment unit 30, a filter 40, a throttle flow path 50, and a switching solenoid valve 60. The pressure adjustment unit 30 includes a pressure adjusting valve 82, and a pilot pressure adjusting solenoid valve 84 for the pressure adjusting valve 82. When the main valve of the pressure adjusting valve 82 opens with an increase in the pilot pressure adjusted by the pilot pressure adjusting solenoid valve 84, the pressure adjusting valve 82 adjusts the pressure of the gas from the gas supply source in accordance with the pilot pressure. The gas whose pressure has been adjusted flows through a flow path in the pressure adjusting valve 82, flows out from the pressure adjusting valve 82, and flows toward the filter 40 and the throttle flow path 50.

As the pilot pressure decreases, the main valve of the pressure adjusting valve 82 closes. In this case, the flow of the gas flowing from the gas supply source into the pressure adjusting valve 82 is blocked. When an exhaust valve of the pressure adjusting valve 82 opens with a further decrease in the pilot pressure, the gas in the flow path in the pressure adjusting valve 82 is discharged from the pressure adjusting valve 82 to the outside of the gas supply device 10.

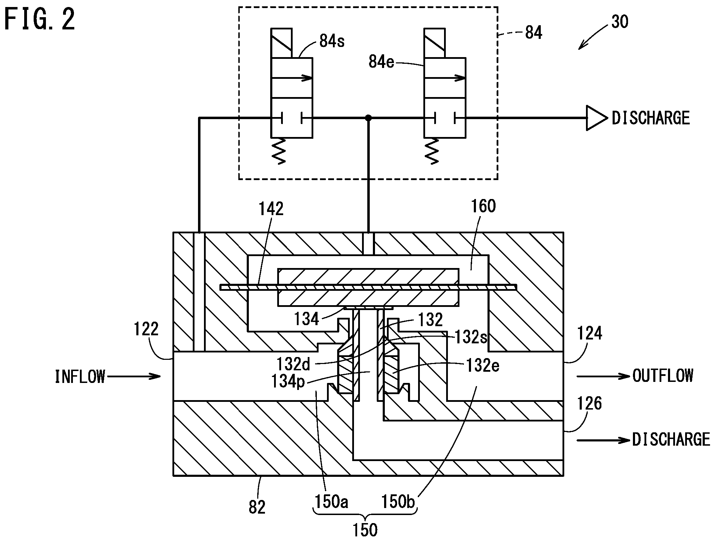

Further, as described later, the gas remaining between the pressure adjusting valve 82 and the switching solenoid valve 60 is also discharged from the pressure adjusting valve 82 to the outside of the gas supply device 10 by opening of the exhaust valve of the pressure adjusting valve 82. The structure of the pressure adjusting valve 82 and the discharge of the gas remaining between the pressure adjusting valve 82 and the switching solenoid valve 60 will be described later with reference to FIG. 2.

As described above, the pilot pressure adjusting solenoid valve 84 adjusts the pilot pressure of the pressure adjusting valve 82. The pilot pressure adjusting solenoid valve 84 includes a supply solenoid valve 84s and an exhaust solenoid valve 84e. The control circuit 20 controls the supply solenoid valve 84s to cause the supply solenoid valve 84s to supply the gas to a pilot chamber of the pressure adjusting valve 82. As a result, the pilot pressure increases. In the example shown in FIG. 1, the supply solenoid valve 84s supplies, to the pilot chamber, a portion of the gas flowing from the gas supply source into the pressure adjusting valve 82. The supply solenoid valve 84s may supply a gas from another gas supply source to the pilot chamber.

The control circuit 20 controls the exhaust solenoid valve 84e to cause the exhaust solenoid valve 84e to discharge the gas from the pilot chamber of the pressure adjusting valve 82. As a result, the pilot pressure decreases. In the example shown in FIG. 1, the supply solenoid valve 84s discharges the gas in the pilot chamber to the outside of the gas supply device 10. Note that the gas in the pilot chamber may be discharged to the outside of the gas supply device 10 together with the gas discharged by the exhaust valve of the pressure adjusting valve 82.

The gas flowing out from the pressure adjusting valve 82 flows through a connecting flow path 90 extending from the pressure adjusting valve 82 to the throttle flow path 50, and flows toward the filter 40 and the throttle flow path 50. The filter 40 is disposed in the connecting flow path 90 and removes dust from the gas flowing through the connecting flow path 90. This prevents the throttle flow path 50 having a narrow flow path width from being clogged.

The gas supply device 10 includes one or a plurality of throttle flow paths 50 downstream of the pressure adjusting valve 82 and the filter 40. In the example shown in FIG. 1, five throttle flow paths 50a, 50b, 50c, 50d, and 50e are provided. Each throttle flow path 50 limits the flow rate of the gas flowing out from the pressure adjusting valve 82 to a predetermined flow rate within a predetermined range corresponding to the throttle flow path 50. In the present embodiment, the predetermined range corresponding to each throttle flow path 50 is included in a throttle range from 0.01 [mL/min] to 1000 [mL/min] corresponding to a minute flow rate.

The predetermined ranges respectively corresponding to the five throttle flow paths 50a, 50b, 50c, 50d, and 50e are different from each other. For example, the throttle flow path 50a limits the flow rate of the gas to a predetermined flow rate within a predetermined range from 0.01 [mL/min] to 0.1 [mL/min] included in the above-described throttle range. The throttle flow path 50b limits the flow rate of the gas to a predetermined flow rate within a predetermined range from 0.1 [mL/min] to 1 [mL/min] included in the above-described throttle range. The throttle flow path 50c limits the flow rate of the gas to a predetermined flow rate within a predetermined range from 1 [mL/min] to 10 [mL/min] included in the above-described throttle range.

The throttle flow path 50d limits the flow rate of the gas to a predetermined flow rate within a predetermined range from 10 [mL/min] to 100 [mL/min] included in the above-described throttle range. The throttle flow path 50e limits the flow rate of the gas to a predetermined flow rate within a predetermined range from 100 [mL/min] to 1000 [mL/min] included in the above-described throttle range. The gas is output by any one of the throttle flow paths 50 at a predetermined flow rate within a predetermined range corresponding to the throttle flow path 50, as the gas to be supplied to the biological sample.

The specific predetermined flow rate can take any value based on the pressure of the gas that is adjusted according to the pilot pressure of the pressure adjusting valve 82 described above. That is, in order to realize the gas supply at a predetermined flow rate, first, the throttle flow path 50 corresponding to the predetermined range including the predetermined flow rate is determined. Next, by appropriately setting the pilot pressure of the pressure adjusting valve 82, the gas is output from the determined throttle flow path 50 at the predetermined flow rate. By increasing the pilot pressure, the predetermined flow rate can be increased.

The connecting flow path 90 includes, on the downstream side of the filter 40, five connecting flow paths 90a, 90b, 90c, 90d, and 90e that branch toward the five throttle flow paths 50. The five connecting flow paths 90a, 90b, 90c, 90d, 90e are connected to the five throttle flow paths 50a, 50b, 50c, 50d, and 50e, respectively.

The switching solenoid valve 60 is connected to the throttle flow path 50 and outputs the gas from the throttle flow path 50. The gas supply device 10 includes one switching solenoid valve 60 or a plurality of switching solenoid valves 60 according to the number of the throttle flow paths 50 to which the switching solenoid valves 60 are connected. In the case where the gas supply device 10 includes a plurality of throttle flow paths 50, the switching solenoid valve 60 outputs the gas from any one of the throttle flow paths 50. Note that, in the case where only one throttle flow path 50 is provided, one switching solenoid valve 60 may be provided or the switching solenoid valve 60 may not be provided. In the example shown in FIG. 1, five switching solenoid valves 60a, 60b, 60c, 60d, and 60e are provided.

Each switching solenoid valve 60 is disposed downstream of the throttle flow path 50 connected thereto, and switches whether or not to output the gas from the throttle flow path 50. The control circuit 20 can control each switching solenoid valve 60 to switch the switching solenoid valve 60 from a valve-open state to a valve-closed state or from the valve-closed state to the valve-open state.

The switching solenoid valve 60 outputs the gas from the throttle flow path 50 by being opened. The gas flows through a flow path segment 100 extending from the throttle flow path 50 to the switching solenoid valve 60, flows through a flow path in the switching solenoid valve 60, and flows out from the gas supply device 10 toward the bioreactor. The switching solenoid valve 60 blocks the output of the gas from the throttle flow path 50 by being closed.

The switching solenoid valve 60 is connected to the throttle flow path 50 via the flow path segment 100. As shown in FIG. 1, five flow path segments 100a, 100b, 100c, 100d, and 100e are shown as the flow path segment 100.

That is, the switching solenoid valve 60a is connected to the throttle flow path 50a via the flow path segment 100a. The switching solenoid valve 60b is connected to the throttle flow path 50b via the flow path segment 100b. The switching solenoid valve 60c is connected to the throttle flow path 50c via the flow path segment 100c. The switching solenoid valve 60d is connected to the throttle flow path 50d via the flow path segment 100d. The switching solenoid valve 60e is connected to the throttle flow path 50e via the flow path segment 100e.

Note that an output flow path 110 includes, on the downstream side of the five switching solenoid valves 60, five output flow paths 110a, 110b, 110c, 110d, and 110e connected to the five switching solenoid valves 60, respectively. The five output flow paths 110a, 110b, 110c, 110d, and 110e merge into one. The gas that has flowed through the merged output flow path 110 is output from the gas supply device 10.

In the case where one switching solenoid valve 60 is in the valve-open state and the remaining switching solenoid valves 60 are in the valve-closed state, the gas can be output from the throttle flow path 50 to which the switching solenoid valve 60 in the valve-open state is connected. The gas output from the throttle flow path 50 flows through the switching solenoid valve 60 in the valve-open state and the output flow path 110, and is output from the gas supply device 10. The gas output from the gas supply device 10 is supplied to the bioreactor.

For example, in the case where the switching solenoid valve 60a is in the valve-open state and the remaining switching solenoid valves 60b, 60c, 60d, and 60e are in the valve-closed state, the gas can be output from the throttle flow path 50a. In this case, the gas from the throttle flow path 50a flows through the switching solenoid valve 60a and the output flow path 110a and is output. In the case where the output of the gas from the throttle flow path 50a is stopped and the output of the gas from the throttle flow path 50b is started, the following processing is performed.

First, the control circuit 20 controls the switching solenoid valve 60a to switch the switching solenoid valve 60a from the valve-open state to the valve-closed state. As a result, the output of the gas from the throttle flow path 50a is stopped. Next, the control circuit 20 controls the switching solenoid valve 60b to switch the switching solenoid valve 60b from the valve-closed state to the valve-open state. As a result, the output of the gas from the throttle flow path 50b is started. Since the switching solenoid valve 60 is provided for each throttle flow path 50 from which the gas is output, it is easy to control the output of the gas whose flow rate is limited to the predetermined flow rate.

In this manner, the switching solenoid valve 60 can output the gas whose flow rate is limited to the predetermined flow rate from any one of the throttle flow paths 50. That is, the switching solenoid valve 60 can switch the predetermined flow rate in the throttle range from 0.01 [mL] to 1000 [mL] and output the gas at the switched predetermined flow rate. Accordingly, the flow rate of the gas can be easily switched to the predetermined flow rate suitable for being supplied to the biological sample.

FIG. 2 is a diagram schematically showing the pressure adjustment unit 30. The pressure adjusting valve 82 of the pressure adjustment unit 30 includes an inlet port 122, an outlet port 124, a discharge port 126, a main valve 132, an exhaust valve 134, and a diaphragm 142. The gas from the gas supply source flows into the inlet port 122.

In the case where the main valve 132 of the pressure adjusting valve 82 is open, a flow path 150 allowing communication between the inlet port 122 and the outlet port 124 is formed. The gas that has flowed into the inlet port 122 flows through the flow path 150 in the pressure adjusting valve 82 and flows out from the outlet port 124. The flow path 150 includes an inflow path 150a from the inlet port 122 to the main valve 132, and an outflow path 150b from the main valve 132 to the outlet port 124. The gas flowing out from the outlet port 124 flows toward the filter 40 and the throttle flow path 50 at a pressure adjusted according to the opening degree of the main valve 132.

The main valve 132 adjusts the pressure of the gas flowing through the flow path 150 according to the pilot pressure adjusted by the pilot pressure adjusting solenoid valve 84. In FIG. 2, both the main valve 132 and the exhaust valve 134 are closed.

In the case where a valve element (valve disc) 132d of the main valve 132 is in contact with a valve seat 132s by being pressed by the repulsive force of an elastic body 132e, the main valve 132 is closed. As described later, when the main valve 132 is pressed by the diaphragm 142 and the valve element 132d is separated from the valve seat 132s, the main valve 132 opens. In the case where the main valve 132 is open, the gas that has flowed into the inlet port 122 flows out from the outlet port 124.

In the case where the exhaust valve 134 is in contact with the main valve 132 by being pressed by the diaphragm 142, the exhaust valve 134 is closed. As described later, when the diaphragm 142 pulls the exhaust valve 134 and the exhaust valve 134 is separated from the main valve 132, the exhaust valve 134 opens. In the case where the exhaust valve 134 is open, the gas in the pressure adjusting valve 82 flows through an exhaust flow path 134p formed in the main valve 132 and is discharged from the discharge port 126.

The supply solenoid valve 84s supplies a gas to a pilot chamber 160 in the pressure adjusting valve 82. The diaphragm 142 is disposed in the pilot chamber 160. As the gas in the pilot chamber 160 increases, the pilot pressure increases. In response to the pilot pressure, the diaphragm 142 pushes the main valve 132. As a result, the main valve 132 opens. The pressure of the gas flowing through the flow path 150 is adjusted according to the opening degree of the main valve 132 corresponding to the pilot pressure.

The exhaust solenoid valve 84e causes the gas in the pilot chamber 160 of the pressure adjusting valve 82 to be discharged to the outside of the gas supply device 10. As the gas in the pilot chamber 160 decreases, the pilot pressure decreases and the diaphragm 142 returns. When the diaphragm 142 is pulled back due to the decrease in the pilot pressure and the main valve 132 of the pressure adjusting valve 82 is closed, the flow of the gas flowing into the inlet port 122 from the gas supply source is blocked. In the case where the inlet port 122 is open, the gas from the gas supply source stops at the inflow path 150a. In the case where the inlet port 122 is closed, the gas from the gas supply source stops outside the inlet port 122.

When the pilot pressure further decreases, the diaphragm 142 pulls the exhaust valve 134 of the pressure adjusting valve 82 according to the pilot pressure. As a result, the exhaust valve 134 opens. According to the pilot pressure, the exhaust valve 134 causes the gas in the flow path 150 and the like to be discharged to the outside of the gas supply device 10 from the discharge port 126.

In the case where the exhaust valve 134 is open, the gas in the flow path 150, the gas in the connecting flow path 90, the gas in the throttle flow path 50, and the gas in the flow path segment 100 are discharged from the discharge port 126. In the case where the inlet port 122 is open, the gas in the outflow path 150b, which is a portion of the gas in the flow path 150, is discharged. When the pressure in each flow path is reduced to the atmospheric pressure, the discharge of the gas is completed. Note that the gas supplied to the biological sample is usually not problematic even if it is released into the atmosphere.

Since the pressure adjusting valve 82 includes the exhaust valve 134, the gas remaining in the flow path 150 in a relatively large amount and the gas in the connecting flow path 90 are discharged without being output from the throttle flow path 50 where the flow rate is limited to a minute flow rate. Therefore, the gas can be discharged in a short time. That is, the gas remaining in the gas supply device 10 can be quickly output.

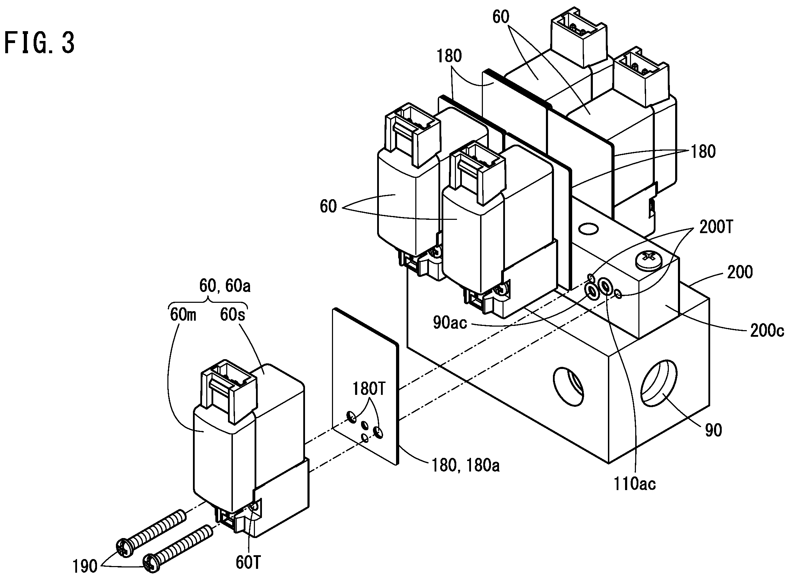

FIG. 3 is a diagram showing a throttle laminated plate 180. In the example shown in FIG. 3, five throttle laminated plates 180 and five switching solenoid valves 60 are each fixed to a protruding portion 200c of a flow path block 200 by a fixing member 190. The fixing member 190 is, for example, a screw. In the example shown in FIG. 3, two screws are used as the fixing member 190. Each switching solenoid valve 60 includes a through hole 60T through and into which the fixing member 190 is inserted and screwed. Each throttle laminated plate 180 includes a through hole 180T through which the fixing member 190 is inserted. A groove 200T into which the fixing member 190 is screwed is formed in the protruding portion 200c of the flow path block 200.

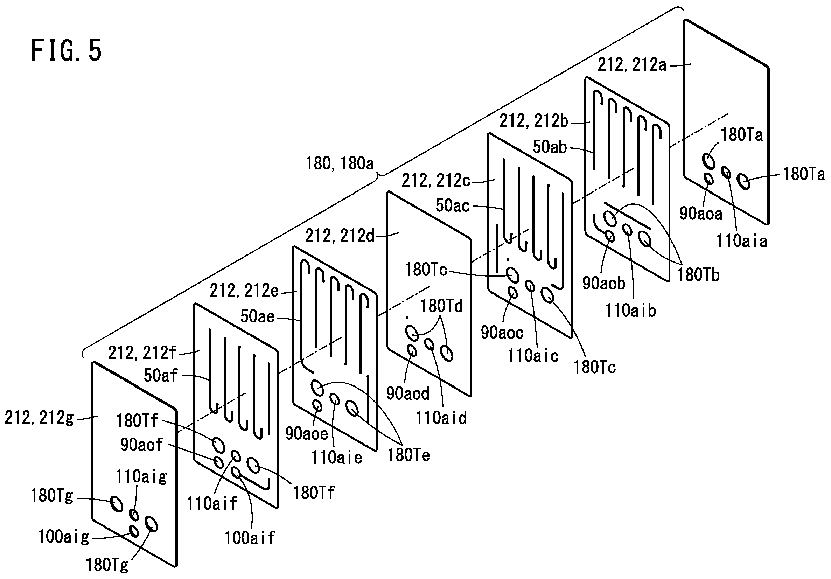

Each throttle flow path 50 is formed inside each throttle laminated plate 180. As described later with reference to FIG. 5, the throttle laminated plate 180 is obtained by laminating a plurality of metal thin plates. The connecting flow path 90 is formed in the flow path block 200. The filter 40 disposed in the connecting flow path 90 is provided in the flow path block 200. The connecting flow paths 90a, 90b, 90c, 90d, and 90e, which are five branches of the connecting flow path 90, are formed in the protruding portion 200c of the flow path block 200. The throttle laminated plate 180 is sandwiched between the switching solenoid valve 60 and the flow path block 200.

The switching solenoid valve 60 includes a main body portion 60m and a solenoid portion 60s. Note that the main body portion 60m of the switching solenoid valve 60 accommodates a valve disposed in the gas flow path in the switching solenoid valve 60. The solenoid portion 60s of the switching solenoid valve 60 opens and closes the valve disposed in the main body portion 60m. The throttle laminated plate 180 is sandwiched between the solenoid portion 60s of the switching solenoid valve 60 and the protruding portion 200c of the flow path block 200.

The connecting flow path 90 branches into five in the flow path block 200. The connecting flow path 90a, which is one of the five connecting flow paths into which the connecting flow path 90 branches, includes an outflow port 90ac formed in the protruding portion 200c of the flow path block 200. The gas flowing through the connecting flow path 90a, which is a portion of the gas that has flowed out from the outlet port 124 of the pressure adjusting valve 82 and passed through the filter 40, reaches the outflow port 90ac.

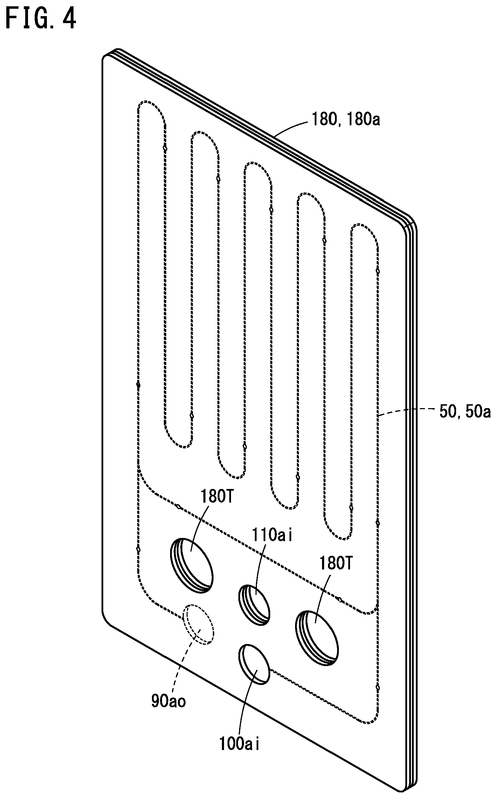

FIG. 4 is an enlarged view of a throttle laminated plate 180a. The throttle laminated plate 180a includes a recess 90ao formed in one surface of the throttle laminated plate 180a that faces the flow path block 200. The gas that has reached the outflow port 90ac flows into the recess 90ao. The recess 90ao is formed at a position where it overlaps the outflow port 90ac. That is, the recess 90ao corresponds to the downstream end of the connecting flow path 90a whose upstream end is the branch point. The upstream end of the throttle flow path 50a is formed in a part of the peripheral wall of the recess 90ao.

The throttle laminated plate 180a includes a recess 100ai formed in the other surface of the throttle laminated plate 180a that faces the switching solenoid valve 60. The gas that has flowed through the throttle flow path 50a flows into the recess 100ai. The downstream end of the throttle flow path 50a is formed in a part of the peripheral wall of the recess 100ai. The recess 100ai corresponds to the upstream end of the flow path segment 100a. The gas that has reached the recess 100ai flows through the flow path segment 100a to the downstream end of the flow path segment 100a formed in the main body portion 60m of the switching solenoid valve 60a.

The gas that has flowed from the downstream end of the flow path segment 100a into the main body portion 60m of the switching solenoid valve 60a flows into the output flow path 110a in the case where the switching solenoid valve 60a is in the valve-open state. The output flow path 110a includes a through hole 110ai of the throttle laminated plate 180a and an inflow port 110ac formed in the protruding portion 200c of the flow path block 200. The gas that has flowed through the through hole 110ai and flowed into the flow path block 200 from the inflow port 110ac flows through the output flow path 110 including the output flow path 110a and is output from the gas supply device 10.

FIG. 5 is a diagram showing a configuration of the throttle laminated plate 180. The throttle laminated plate 180 is obtained by laminating a plurality of metal thin plates 212. The plurality of laminated metal thin plates 212 are integrated by thermal diffusion bonding to form the throttle laminated plate 180. FIG. 5 shows the throttle laminated plate 180 in which seven metal thin plates 212a, 212b, 212c, 212d, 212e, 212f, and 212g are laminated in this order. The metal thin plate 212a faces the flow path block 200. The metal thin plate 212g faces the switching solenoid valve 60.

The above-described recess 90ao is formed of a circular through hole 90aoa of the metal thin plate 212a, a circular through hole 90aob of the metal thin plate 212b, a circular through hole 90aoc of the metal thin plate 212c, a circular through hole 90aod of the metal thin plate 212d, a circular through hole 90aoe of the metal thin plate 212e, a circular through hole 90aof of the metal thin plate 212f, and the metal thin plate 212g.

That is, the metal thin plate 212g having no through hole at a position where the through holes 90aoa, 90aob, 90aoc, 90aod, 90aoe, and 90aof overlap each other is laminated on the metal thin plates 212a, 212b, 212c, 212d, 212e, and 212f. As a result, the recess 90ao is formed.

The metal thin plate 212b further includes a through hole 50ab having a straight and curved shape. The metal thin plates 212a and 212c, which have no through hole at the position of the through hole 50ab, are laminated with the metal thin plate 212b interposed therebetween. As a result, a part of the throttle flow path 50a is formed. In the metal thin plate 212b, the through hole 50ab having a straight and curved shape is connected to the circular through hole 90aob. As a result, the above-described upstream end of the throttle flow path 50a is formed.

The metal thin plate 212c further includes a through hole 50ac having a straight and curved shape. The metal thin plates 212b and 212d, which have no through hole at the position of the through hole 50ac, are laminated with the metal thin plate 212c interposed therebetween. As a result, a part of the throttle flow path 50a is formed. The metal thin plate 212e further includes a through hole 50ae having a straight and curved shape. The metal thin plates 212d and 212f, which have no through hole at the position of the through hole 50ae, are laminated with the metal thin plate 212e interposed therebetween. As a result, a part of the throttle flow path 50a is formed.

The metal thin plate 212f further includes a through hole 50af having a straight and curved shape. The metal thin plates 212e and 212g, which have no through hole at the position of the through hole 50af, are laminated with the metal thin plate 212f interposed therebetween. As a result, a part of the throttle flow path 50a is formed.

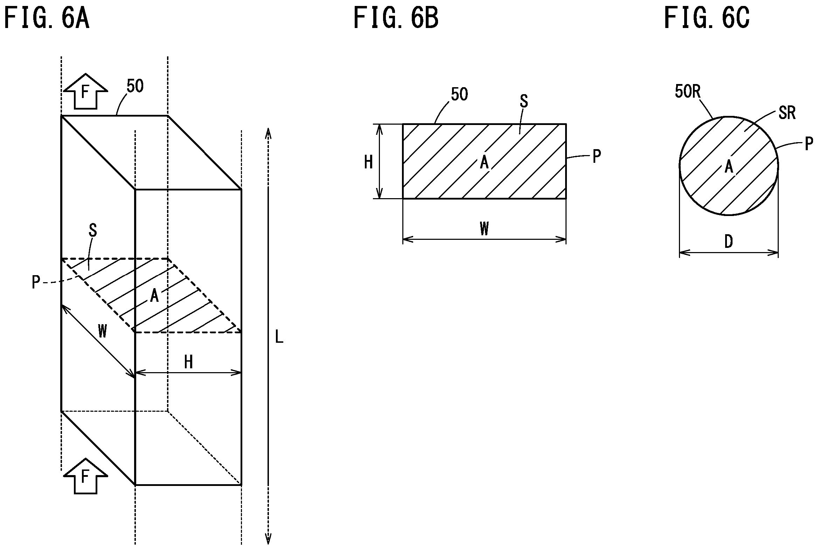

By laminating the metal thin plates 212a, 212b, 212c, 212d, 212e, 212f, and 212g, the throttle flow path 50a having a rectangular cross section is formed with a flow path width D and a flow path length L corresponding to the above-described predetermined flow rate. By appropriately designing the flow path width D and the flow path length L, the predetermined flow rate is easily realized. The calculation of the flow path width D in the rectangular cross section perpendicular to the flow direction of the gas flowing through the throttle flow path 50a will be described later with reference to FIGS. 6A, 6B, and 6C. Note that the predetermined flow rate changes according to the pilot pressure of the pressure adjusting valve 82. The other throttle flow paths 50b, 50c, 50d, and 50e are formed in the same manner as the throttle flow path 50a.

The above-described recess 100ai is formed of the metal thin plate 212e, a circular through hole 100aif of the metal thin plate 212f, and a circular through hole 100aig of the metal thin plate 212g. That is, the metal thin plate 212e having no through hole at a position where the through hole 100aif and the through hole 100aig overlap each other is laminated on the metal thin plates 212f and 212g. As a result, the recess 100ai is formed.

The above-described through hole 110ai is formed of a circular through hole 110aia of the metal thin plate 212a, a circular through hole 110aib of the metal thin plate 212b, a circular through hole 110aic of the metal thin plate 212c, a circular through hole 110aid of the metal thin plate 212d, a circular through hole 110aie of the metal thin plate 212e, a circular through hole 110aif of the metal thin plate 212f, and a circular through hole 110aig of the metal thin plate 212g.

That is, the metal thin plates 212a, 212b, 212c, 212d, 212e, 212f, and 212g are laminated so that the through holes 110aia, 110aib, 110aic, 110aid, 110aie, 110aif, and 110aig overlap each other. As a result, the through hole 110ai is formed.

The above-described through hole 180T is formed of a circular through hole 180Ta of the metal thin plate 212a, a circular through hole 180Tb of the metal thin plate 212b, a circular through hole 180Tc of the metal thin plate 212c, a circular through hole 180Td of the metal thin plate 212d, a circular through hole 180Te of the metal thin plate 212e, a circular through hole 180Tf of the metal thin plate 212f, and a circular through hole 180Tg of the metal thin plate 212g.

That is, the metal thin plates 212a, 212b, 212c, 212d, 212e, 212f, and 212g are laminated so that the through holes 180Ta, 180Tb, 180Tc, 180Td, 180Te, 180Tf, and 180Tg overlap each other. As a result, the through hole 180T is formed.

As described above, the fixing member 190 is inserted through the through hole 180T of the throttle laminated plate 180 sandwiched between the switching solenoid valve 60 and the protruding portion 200c of the flow path block 200. The fixing member 190 is screwed into the through hole 60T of the switching solenoid valve 60 and the groove 200T of the protruding portion 200c. That is, the switching solenoid valve 60 and the throttle laminated plate 180 including the throttle flow path 50 to which the switching solenoid valve 60 is connected are fastened together by the fixing member 190 and fixed to the flow path block 200.

In the example shown in FIG. 3, the solenoid portion 60s of the switching solenoid valve 60 is fixed to the protruding portion 200c of the flow path block 200 together with the throttle laminated plate 180 sandwiched therebetween. Specifically, the main body portion 60m of the switching solenoid valve 60, the solenoid portion 60s of the switching solenoid valve 60, the throttle laminated plate 180, and the protruding portion 200c of the flow path block 200 are arranged in this order and fixed to each other. As a result, the rigidity of the relatively thin throttle laminated plate 180 can be ensured.

By fastening together the switching solenoid valve 60 and the throttle laminated plate 180, the length of the flow path segment 100 from the throttle flow path 50 to the switching solenoid valve 60 can be reduced. As a result, the gas supply device 10 can be downsized. In the case where the flow path segment 100 is long, a large amount of gas remains in the flow path segment 100 after each switching solenoid valve 60 is brought into the valve-closed state in order to stop the supply of the gas from the gas supply device 10 to the biological sample.

In the case where the exhaust valve 134 of the pressure adjusting valve 82 is open, the gas in the flow path 150, the gas in the connecting flow path 90, the gas in the throttle flow path 50, and the gas in the flow path segment 100 are discharged from the discharge port 126. However, since the flow rate of the gas is limited to a minute flow rate by the throttle flow path 50, it takes a long time until the discharge of the gas in the flow path segment 100 is completed. Note that, when the switching solenoid valve 60 is brought into the valve-open state, the gas in the flow path segment 100 can be output from the output flow path 110. However, in that case, an overshoot may occur in which a large amount of high pressure gas is output to the bioreactor. This may cause the biological sample to die or the like.

In the present embodiment, since the length of the flow path segment 100 can be reduced, the time until the discharge of the gas in the flow path segment 100 is completed can be shortened.

FIG. 6A is a diagram explaining a rectangular cross section S of the throttle flow path 50. The rectangular cross section S is a cut surface obtained in the case where the throttle flow path 50 is cut perpendicularly to a flow direction F of the gas flowing through the throttle flow path 50a having the flow path length L. FIG. 6A illustrates a width W of the rectangular cross section S, a height H of the rectangular cross section S, a perimeter P of the rectangular cross section S, and an area A of the rectangular cross section S.

FIG. 6B is a diagram showing the rectangular cross section S of the throttle flow path 50. The perimeter P of the rectangular cross section S is expressed by Expression (1) based on the width W of the rectangular cross section S and the height H of the rectangular cross section S. The area A of the rectangular cross section S is expressed by Expression (2) based on the width W of the rectangular cross section S and the height H of the rectangular cross section S.

$$P = 2 \cdot (W + H) \ldots (1)$$

$$A = W \cdot H \ldots (2)$$

FIG. 6C is a diagram explaining the flow path width D of the throttle flow path 50. FIG. 6C shows a circular cross section SR of an equivalent flow path 50R that is equivalent to the throttle flow path 50. In general, the diameter of the circular cross section SR having a perimeter P and an area A that are respectively identical to the perimeter P and the area A of the rectangular cross section S is known as an equivalent diameter of the rectangular cross section S. In the present embodiment, the equivalent diameter of the rectangular cross section S is used as the flow path width D of the throttle flow path 50. Therefore, the flow path width D of the throttle flow path 50 is expressed by Expression (3). Using Expressions (1), (2), and (3), the flow path width D of the throttle flow path 50 is expressed by Expression (4).

$$D = 4 \cdot A/P \ldots (3)$$

$$D = 4 \cdot W \cdot H/(2 \cdot (W + H)) \ldots (4)$$

The predetermined flow rate limited by each throttle flow path 50 is determined according to the flow path width D and the flow path length L of the throttle flow path 50 and the pilot pressure of the pressure adjusting valve 82. That is, the flow path width D and the flow path length L of the throttle flow path 50 are determined for each throttle flow path 50. As the flow path width D decreases, the influence by the inner wall of the throttle flow path 50 increases, and therefore, the predetermined flow rate decreases. By appropriately designing the flow path width D, it is possible to create the throttle flow path 50 capable of outputting a gas at a minute flow rate suitable for being supplied to the biological sample.

Further, as the flow path length L increases, the influence by the inner wall of the throttle flow path 50 increases, and therefore, the predetermined flow rate decreases. By appropriately designing the flow path length L, it is possible to create the throttle flow path 50 capable of outputting a gas at a minute flow rate suitable for being supplied to the biological sample. In the case where all the throttle flow paths 50 have the same flow path width D, the flow path length L of each throttle flow path 50 can be designed using a value obtained by multiplying the flow path width D by N.

For example, N = 7000 is used for designing the flow path length L of the throttle flow path 50a where the predetermined flow rate is the lowest. That is, the flow path length L of the throttle flow path 50a is designed to satisfy L = 7000 · D. Accordingly, the flow rate of the gas output from the throttle flow path 50a can be limited to a predetermined flow rate included in a predetermined range from 0.01 [mL/min] to 0.1 [mL/min].

For example, N = 500 is used for designing the flow path length L of the throttle flow path 50e where the predetermined flow rate is the highest. That is, the flow path length L of the throttle flow path 50e is designed to satisfy L = 500 · D. Accordingly, the flow rate of the gas output from the throttle flow path 50e can be limited to a predetermined flow rate included in a predetermined range from 100 [mL/min] to 1000 [mL/min].

As described above, the throttle flow path 50 outputs the gas at the predetermined flow rate as the gas to be supplied to the biological sample. Therefore, the flow path length L of the throttle flow path 50 preferably has a value obtained by multiplying the flow path width D by at least 500. As a result, the gas is output from the throttle flow path 50 at the predetermined flow rate suitable for being supplied to the biological sample.

The following supplementary notes are further disclosed in relation to the above disclosure.

(Supplementary Note 1)

The gas supply device (10) that performs gas supply to the biological sample includes: the pressure adjusting valve (82) including the inlet port (122) into which a gas from the gas supply source flows, the outlet port (124) from which the gas flows out, the discharge port (126) from which the gas is discharged to the outside of the gas supply device, the main valve (132) configured to adjust, according to the pilot pressure, the pressure of the gas flowing through the flow path (150) configured to allow communication between the inlet port and the outlet port, and the exhaust valve (134) configured to discharge the gas in the flow path from the discharge port according to the pilot pressure; the pilot pressure adjusting solenoid valve (84) configured to adjust the pilot pressure; and the throttle flow path (50) configured to limit the flow rate of the gas flowing out from the outlet port to the predetermined flow rate and outputs the gas at the predetermined flow rate for the gas supply. According to such a configuration, the gas remaining in the gas supply device can be quickly output.

(Supplementary Note 2)

In the gas supply device according to Supplementary Note 1, the throttle flow path may be formed in the throttle laminated plate (180) in which the metal thin plates (212) are laminated, the throttle flow path having the flow path width (D)

and the flow path length (L) that correspond to the predetermined flow rate. According to such a configuration, a predetermined flow rate is easily realized.

(Supplementary Note 3)

In the gas supply device according to Supplementary Note 2, the predetermined flow rate may decrease as the flow path length increases. According to such a configuration, by appropriately designing the flow path length, it is possible to create the throttle flow path capable of outputting a gas at a minute flow rate suitable for being supplied to the biological sample.

(Supplementary Note 4)

In the gas supply device according to Supplementary Note 2, the predetermined flow rate may decrease as the flow path width decreases. According to such a configuration, by appropriately designing the flow path width, it is possible to create the throttle flow path capable of outputting a gas at a minute flow rate suitable for being supplied to the biological sample.

(Supplementary Note 5)

In the gas supply device according to Supplementary Note 2, the flow path length may have a value obtained by multiplying the flow path width by at least 500. According to such a configuration, the gas is output from the throttle flow path at the predetermined flow rate suitable for being supplied to the biological sample.

(Supplementary Note 6)

In the gas supply device according to any one of Supplementary Notes 1 to 5, the pas supply device may include a plurality of the throttle flow paths, the predetermined flow rates of the plurality of throttle channels are different from each other, and the gas supply device may further include the switching solenoid valve (60) connected to the plurality of throttle flow paths and configured to output the gas from any one of the throttle flow paths. According to such a configuration, the flow rate of the gas can be easily switched to the predetermined flow rate suitable for being supplied to the biological sample.

(Supplementary Note 7)

In the gas supply device according to Supplementary Note 6, the gas supply device may include a plurality of the switching solenoid valves, and the plurality of switching solenoid valves may be respectively connected to the plurality of throttle flow paths. According to such a configuration, it is easy to control the output of the gas whose flow rate is limited to the predetermined flow rate.

(Supplementary Note 8)

In the gas supply device according to Supplementary Note 6, by outputting the gas from any one of the throttle flow paths, the switching solenoid valve may switch the predetermined flow rate in a throttle range from 0.01 [mL] to 1000 [mL], and output the gas at the predetermined flow rate that has been switched. According to such a configuration, the flow rate of the gas can be easily switched to the predetermined flow rate suitable for being supplied to the biological sample.

(Supplementary Note 9)

The gas supply device according to any one of Supplementary Notes 2 to 5 may further include the switching solenoid valve connected to the throttle flow path and configured to output the gas from the throttle flow path, wherein the switching solenoid valve and the throttle laminated plate may be fastened together. According to such a configuration, it is possible to shorten the time until the discharge of the gas in the flow path segment extending from the throttle flow path to the switching solenoid valve is completed.

(Supplementary Note 10)

The gas supply device according to any one of Supplementary Notes 1 to 5 may further include the filter (40) that is disposed in the connecting flow path (90) which extends from the outlet port to the throttle flow path and through which the gas flows, the filter being configured to remove dust from the gas. According to such a configuration, it is possible to prevent the throttle flow path having a narrow flow path width from being clogged.

The present invention is not limited to the above disclosure, and various modifications are possible without departing from the essence and gist of the present invention.

## Claims

1.  A gas supply device (10) that performs gas supply to a biological sample, the gas supply device comprising:

    a pressure adjusting valve (82) including an inlet port (122) into which a gas from a gas supply source flows, an outlet port (124) from which the gas flows out, a discharge port (126) from which the gas is discharged to an outside of the gas supply device, a main valve (132) configured to adjust, according to a pilot pressure, a pressure of the gas flowing through a flow path (150) configured to allow communication between the inlet port and the outlet port, and an exhaust valve (134) configured to discharge the gas in the flow path from the discharge port according to the pilot pressure;
    a pilot pressure adjusting solenoid valve (84) configured to adjust the pilot pressure; and
    a throttle flow path (50) configured to limit a flow rate of the gas flowing out from the outlet port to a predetermined flow rate and output the gas at the predetermined flow rate for the gas supply.

2.  The gas supply device according to claim 1, wherein
    the throttle flow path is formed in a throttle laminated plate (180) in which metal thin plates (212) are laminated, the throttle flow path having a flow path width (D) and a flow path length (L) that correspond to the predetermined flow rate.

3.  The gas supply device according to claim 2, wherein
    the predetermined flow rate decreases as the flow path length increases.

4.  The gas supply device according to claim 2, wherein
    the predetermined flow rate decreases as the flow path width decreases.

5.  The gas supply device according to claim 2, wherein
    the flow path length has a value obtained by multiplying the flow path width by at least 500.

6.  The gas supply device according to any one of claims 1 to 5, wherein

    the gas supply device comprises a plurality of the throttle flow paths,
    the predetermined flow rates of the plurality of throttle flow paths are different from each other, and
    the gas supply device further comprises a switching solenoid valve (60) connected to the plurality of throttle flow paths and configured to output the gas from any one of the throttle paths.

7.  The gas supply device according to claim 6, wherein

    the gas supply device comprises a plurality of the switching solenoid valves, and
    the plurality of switching solenoid valves are respectively connected to the plurality of throttle flow paths.

8.  The gas supply device according to claim 6, wherein
    by outputting the gas from any one of the throttle flow paths, the switching solenoid valve switches the predetermined flow rate in a throttle range from 0.01 [mL] to 1000 [mL], and outputs the gas at the predetermined flow rate that has been switched.

9.  The gas supply device according to any one of claims 2 to 5, further comprising a switching solenoid valve connected to the throttle flow path and configured to output the gas from the throttle flow path, wherein
    the switching solenoid valve and the throttle laminated plate are fastened together.

10. The gas supply device according to any one of claims 1 to 5, further comprising a filter (40) that is disposed in a connecting flow path (90) which extends from the outlet port to the throttle flow path and through which the gas flows, the filter being configured to remove dust from the gas.

# FIG. 1

FIG. 2

30

84

84s    84e

DISCHARGE

INFLOW

OUTFLOW

DISCHARGE

122    142    160    132    132s    124

134    132d    132e    126

134p    150a    150b

150

82

EP 4 756 008 A1

FIG. 3

FIG. 4

180, 180a

50, 50a

180T

110ai

180T

90ao

100ai

FIG. 5

FIG. 6A

FIG. 6B

FIG. 6C

EP 4 756 008 A1

## INTERNATIONAL SEARCH REPORT

| International application No. |
| --- |
| **PCT/JP2024/018077** |

**A. CLASSIFICATION OF SUBJECT MATTER**

*C12M 1/04*(2006.01)i; *G05D 7/06*(2006.01)i
FI:   C12M1/04: G05D7/06 B

According to International Patent Classification (IPC) or to both national classification and IPC

**B. FIELDS SEARCHED**

Minimum documentation searched (classification system followed by classification symbols)

C12M1/04: G05D7/06; F16K

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2005-157644 A (SMC CORP.) 16 June 2005 (2005-06-16) | 1-10 |
| A | JP 2020-113018 A (SMC CORP.) 27 July 2020 (2020-07-27) | 1-10 |
| A | JP 2021-170935 A (CKD CORPORATION) 01 November 2021 (2021-11-01) | 1-10 |
| A | JP 2011-160729 A (AIRTECH JAPAN LTD.) 25 August 2011 (2011-08-25) | 1-10 |
| A | JP 2007-265395 A (SMC CORP.) 11 October 2007 (2007-10-11) | 1-10 |
| A | EP 3978596 A1 (SORBONNE UNIVERSITE) 06 April 2022 (2022-04-06) | 1-10 |
| P, A | JP 2024-021395 A (SMC CORP.) 16 February 2024 (2024-02-16) | 1-10 |

☐ Further documents are listed in the continuation of Box C.     ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 July 2024** | **30 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/018077**

| Patent document cited in search report | | | Publication date (day/month/year) | Patent family member(s) | | | Publication date (day/month/year) |
|---|---|---|---|---|---|---|---|
| JP | 2005-157644 | A | 16 June 2005 | (Family: none) | | | |
| JP | 2020-113018 | A | 27 July 2020 | US | 2020/0224680 | A1 | |
| | | | | EP | 3680744 | A1 | |
| | | | | CN | 111434956 | A | |
| | | | | KR | 10-2020-0087718 | A | |
| | | | | TW | 202034106 | A | |
| JP | 2021-170935 | A | 01 November 2021 | (Family: none) | | | |
| JP | 2011-160729 | A | 25 August 2011 | (Family: none) | | | |
| JP | 2007-265395 | A | 11 October 2007 | US | 2007/0205384 | A1 | |
| | | | | DE | 102007009869 | A1 | |
| | | | | KR | 10-2007-0090843 | A | |
| | | | | CN | 101029652 | A | |
| EP | 3978596 | A1 | 06 April 2022 | US | 2024/0002776 | A1 | |
| | | | | WO | 2022/069662 | A1 | |
| | | | | CN | 116323908 | A | |
| JP | 2024-021395 | A | 16 February 2024 | US | 2024/0044421 | A1 | |
| | | | | EP | 4350153 | A1 | |
| | | | | CN | 117514964 | A | |
| | | | | KR | 10-2024-0019029 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018070464 A1